(19) Europäisches Patentamt
European Patent Office
Office européen des brevets

(11) **EP 0 761 188 A2**

(12) **EUROPÄISCHE PATENTANMELDUNG**

(43) Veröffentlichungstag:
12.03.1997 Patentblatt 1997/11

(51) Int. Cl.⁶: **A61F 13/04**, A61F 5/30,
A61L 15/08

(21) Anmeldenummer: 96112965.7

(22) Anmeldetag: 12.08.1996

(84) Benannte Vertragsstaaten:
AT BE CH DE DK ES FR GB GR IE IT LI LU MC NL PT SE

(30) Priorität: 10.08.1995 DE 19529500

(71) Anmelder: 1st Memory Alloys GmbH
61279 Grävenwiesbach 2 (DE)

(72) Erfinder:
• Steckmann, Helge G.
D-61279 Grävenwiesbach (DE)
• Prieb, Viktor, Dr.
D-12169 Berlin (DE)

(74) Vertreter: KUHNEN, WACKER & PARTNER
Alois-Steinecker-Strasse 22
85354 Freising (DE)

(54) **Verbundgewebe mit Memory-Metall und Anwendungen hiervon**

(57) Es wird ein Verbundgewebe mit Memory-Metall bereitgestellt sowie verschiedene Anwendungen hiervon diskutiert. Hierbei werden in ein herkömmliches flächiges Stoffgewebe Fäden bzw. Drähte (4) aus einer Legierung mit Formgedächtniseigenschaften (Memory-Metall) eingearbeitet. Durch gezielte Wärmebehandlung, zum Beispiel mittels elektrischem Strom, führen die eingearbeiteten Drähte (4) reversibel zu einer Kontraktion des Verbundgewebes, wodurch zum Beispiel ein Massageeffekt erreicht werden kann.

Fig. 2

**Beschreibung**

Die Erfindung betrifft ein Verbundgewebe mit Memory-Metall sowie Anwendungen hiervon in der Medizin, Aero- und Raumfahrttechnik.

Im Bereich der Medizin werden Stütz- bzw. Pressverbände verwendet, um Ödeme bzw. Schwellungen von Gliedmaßen zu behandeln. Für diese Zwecke werden gummiähnliche Stoffe verwendet, die eine konstante Kraft auf die darunterliegende Körperregion ausüben. Ein Nachteil derartiger herkömmlicher Stütz- bzw. Pressverbände und Manschetten besteht darin, daß die ausgeübte Anpresskraft nicht regulierbar ist. Sie müssen in bereits angespanntem Zustand an das betroffene Körperteil angelegt werden, was sich je nach gewünschtem Körperdruck nach dem Anlegen, mehr oder minder schwierig gestaltet.

Bei der Behandlung von postoperativen Ödemen im Zusammenhang mit Frakturen, bei denen Fixaturen notwendig sind, ist die Verwendung von herkömmlichen elastischen Stütz- bzw. Pressverbänden erheblich erschwert bzw. zum Teil unmöglich.

Im Bereich der Sportmedizin und Orthopädie werden auch sog. Gelenkorthesen verwendet, die starre Komponenten aufweisen, die über Gelenkverbindungen miteinander verbunden sind und so das Abbiegen des Knies etc. ermöglichen. Nachteilig bei diesem bekannten Knieorthesen ist, daß sie sehr teuer sind, da sie individuell angepaßt bzw. hergestellt werden müssen. Darüber hinaus leidet aufgrund der starren Elemente der Tragekomfort.

Zur Behandlung von Schwellungen und Ödemen verschiedener Arten ist es bekannt Apparate mit intermittierender Kompression zur Behandlung einzusetzen. Derartiger Geräte, wie sie beispielsweise in dem Sonderdruck 2/90 vom 15. Februar 1990, Der niedergelassene Arzt: Die Therapie des Lymphödems, beschrieben sind umfassen Manschetten mit einzelnen Luftkammern, die zusammen oder unabhängig voneinander mit Druckluft beaufschlagt werden. Ein verbessertes Gerät, das auch Druckgradienten aufbauen kann, ist aus der Firmendruckschrift Vasomed Aktuell, 7. Jahrgang Nr. 2/95 mit dem Titel: Neuester Stand der apparativen intermittierenden Kompression: Das 3-Phasen-Gradient-System, bekannt. Derartige Geräte werden auch von der Firma Bösl Medizintechnik GmbH in 52068 Aachen unter der Bezeichnung "lympha-mat GRADIENT" (12-Kammer-System) und "vasoflow GRADIENT" angeboten. Diese bekannten Massagevorrichtungen für intermittierende Kompression sind vergleichsweise teuer, da ein Druckluftkompressor nötig ist. Darüber hinaus ist die Anwendung bei Brüchen, die durch eine Fixatur gesichert sind, aufgrund des sich ändernden Volumens der Druckluftkammern unmöglich.

Auch im Bereich der Luft- und Raumfahrttechnik werden Pilotendruckanzüge mit Luftkammern verwendet, die bei Bedarf mit Druckluft beaufschlagt werden, um den Blutentzug aus dem Kopfbereich in die Extremitäten zu verhindern. Aufgrund des Volumens der Luftkammern sind diese Druckanzüge vergleichsweise unförmig und die Ansteuerung ist technisch aufwendig, da ebenfalls mit Druckluft gearbeitet wird.

Aus Trendletter 05/94 - Innovationen ist eine Matratze bekannt, die das Wundliegen von bettlägerigen Patienten verhindert. Bei dieser von der japanischen Firma Nippon Tungsten Co. (20-31, Shimizu 2-chome, Minami-ku, Fukuoka 815) entwickelten elektrischen Matratze - ähnlich einer Heizdecke - hebt und senkt sich in regelmäßigen Abständen die Oberfläche der Matratze wellenförmig. Dies wird durch eingewobene dünne Drähte aus einer Shape-Memory-Legierung erreicht, die bei Erreichung einer bestimmten Temperatur ihre Form verändert. Die zeitlichen Intervalle werden mit einem Timer geregelt.

Demgegenuber ist es Aufgabe der vorliegenden Erfindung ein Verbundgewebe mit Memory-Metall anzugeben, das einfach herzustellen und vielseitig verwendbar ist. Weiter ist es Aufgabe der vorliegenden Erfindung einen Press- bzw. Stützverband, eine Massagevorrichtung bzw. eine Vorrichtung für intermittierende Kompression und einen Pilotendruckanzug mit einem derartigen Verbundgewebe anzugeben.

Die Lösung dieser Aufgabe erfolgt durch die Merkmale des Anspruchs 1, 16, 19 bzw. 22.

Dadurch, daß die Fäden bzw. Drähte aus Memory-Metall bzw. einer Legierung mit Formgedächtniseigenschaften in ein flächiges Stoffgewebe eingearbeitet werden, ergibt sich ein flächiges Verbundgewebe, das vielseitig verwendbar ist.

Gemäß der vorteilhaften Ausgestaltung der Erfindung nach Anspruch 2 werden die Drähte aus Memory-Metall zwischen zwei flächigen Schichten aus Stoffgewebe aus Stoffgewebe nach Art eines Sandwich eingearbeitet. Es kann beispielsweise mittels Ultraschall-Schweißen erfolgen. Gemäß der vorteilhaften Ausgestaltung der Erfindung nach Anspruch 3 werden die Memory-Metalldrähte auf ein flächiges Stoffgewebe aufgesteppt. In beiden Fällen ergibt sich eine einfache Herstellung des erfindungsgemäßen Verbundgewebes aus einem herkömmlichen Stoffgewebe und im Handel erhältlichen Drähten aus Memory-Metall.

Bei der Verwendung des erfindungsgemäßen Verbundgewebes in einem Press- bzw. Stützverband nach Anspruch 16 wird die Metallegierung für die Memory-Drähte so ausgewählt, daß die Temperatur $A_S$ bei der die Längenkontraktion der Fäden bei Temperaturerhöhung einsetzt, größer als die durchschnittliche Körpertemperatur des Körperteils ist, an den der Press- bzw. Stützverband angelegt wird. Damit wird erreicht, daß der Press- bzw. Stützverband in entlastetem Zustand angelegt werden kann. Ist der erfindungsgemäße Press- bzw. Stützverband in gewünschter Weise appliziert, so kann durch Erhöhung der Temperatur über die Temperatur $A_S$ hinaus gezielt die Kontraktion und damit die Druckerhöhung eingeleitet werden.

Die Nutzung des Zweiweg-Memory-Effekts ist vorteilhaft, da dadurch ein Massageeffekt mit wiederholter Spannung und Entspannung erzielt werden kann (Anspruch 4). Dies wird in vorteilhafterweise durch ein elastisches Stoffgewebe unterstützt (Anspruch 5).

Durch Drähte mit rundem Querschnitt wird die Verletzungsgefahr minimiert und runde Drähte sind einfach herzustellen (Anspruch 6).

Durch die vorteilhafte Ausgestaltung der Erfindung gemäß den Ansprüchen 7 und 8 wird die maximal erzielbare Kontraktion und damit der maximal ausübbare Druck erhöht.

Durch unterschiedliche Drahtquerschnitte (Anspruch 9) lassen sich die mechanischen Eigenschaften des Verbundgewebes in verschiedenen Flächenabschnitten gezielt beeinflussen und variieren.

Mittels elektrischen Strom (Anspruch 10) läßt sich auf einfache Weise die nötige Temperaturerhöhung herbeiführen. Auch kann damit auf einfache Weise eine Temperturregelung erreicht werden.

Zusätzlich oder alternativ kann die Aufheizung bzw. Temperaturerhöhung induktiv erfolgen. Hierzu werden Stromstöße in geschlossene Drahtschlerfen aus Memorymetall induziert (Anspruch 11). Ein elektrisch isolierendes Stoffgewebe (Anspruch 12) erhöht die Sicherheit bei Aufheizung mittels Strom.

Durch die Ausbildung als Endlosverband bzw. als Verbandsbinde, die zu einer Berbandsrolle aufgewickelt wierd, wird erreicht, daß Verbandmaterial mit dem erfindungsgemäßen Verbundgewebe in gleicherweise angewendet werden kann, wie herkömmliche Verbandsrollen (Anspruch 13) Dies gilt auch für die Ausbildung in Form einer Manschette (Anspruch 14).

Durch die in Anspruch 15 angegeben Materialien bzw. Verbindungen lassen sich die kritischen Temperaturen der Memorymetalle in den gewünschten Temperaturbereichen Verschieben bzw. Einstellen.

Durch die vorteilhafte Ausgestaltung der Erfindung nach den Ansprüchen 16, 17 bzw. 18 wird erreicht, daß der erfindungsgemäße Preß- und Stützverband wie ein herkömmlicher Verband aufgebracht werden kann und dann in angelegtem Zustand die Kontraktion und damit die Druckerhöhung gezielt herbeigeführt werden kann. Durch geeignete Temperaturwahl (Anspruch 17) ist ein sicherer Halt bzw. eine dauerhafte Kontraktion gewährleistet.

Bei der Verwendung des erfindungsgemäßen Verbundgewebes zur Bereitstellung einer Massagevorrichtung bzw. einer Vorrichtung zur Erzeugung von intermittierender Kompression gemäß Anspruch 19 wird die Temperaturhysterese der Memory-Metalllegierung so gewählt, daß sowohl die Temperatur $A_s$, bei der die Längenkontraktion bei Temperaturerhöhung einsetzt, als auch die Temperatur $M_f$, bei der bei Temperaturerniedrigung die Fäden wieder beginnen ihre ursprüngliche Länge einzunehmen, größer als die durchschnittliche Temperatur des zu massierenden Körpers gewählt. Dadurch wird ermöglicht, daß beispielsweise durch periodische Erwärmung der Memory-Metalldrähte mittels kurzen Stromimpulsen eine Kontraktion stattfindet, die dann durch Abschalten des Stromflusses aufgrund der Abkühlung auf Umgebungs- bzw. Körpertemperatur wieder rückgängig gemacht wird.

Weitere Einzelheiten, Merkmale und Vorteile der Erfindung ergeben sich aus der nachfolgenden Beschreibung von bevorzugten Ausführungsformen anhand der Zeichnungen.

Es zeigt:

Fig. 1a und b    Hysteresediagramme von Memory-Metallegierungen, wie sie bei der vorliegenden Erfindung verwendet werden,

Fig. 2    eine schematische Darstellung eines flächigen Stoffgewebes mit darin eingearbeiteten Memory-Metalldrähten,

Fig. 3    eine schematische Darstellung zur Herstellung der mäanderförmig ausgebildeten Memory-Metalldrähte,

Fig. 4a und b    Beispiele zur Einarbeitung der Memory-Metalldrähte in das flächige Stoffgewebe, und

Fig. 5    eine schematische Darstellung einer Massageeinrichtung gemäß der vorliegenden Erfindung.

Man unterscheidet den Einweg-Memory-Effekt und den Zweiweg-Memory-Effekt. Bei dem Einweg-Memory-Effekt wird das Memory-Metall mechanisch verformt und durch Erhitzen des verformten Metallstücks über die Temperatur $A_s$ hinaus wird die Verformung wieder rückgängig gemacht. Eine erneute Abkühlung des Memory-Metalls führt jedoch zu keiner weiteren Formänderung und man spricht daher von einem Einweg-Memory-Effekt. Bei dem Zweiweg-Memory-Effekt wird ein Stück Memory-Metall zunächst so stark verformt, daß ein Teil der Verformung irreversibel ist. Bei der anschließenden Erwärmung über die Temperatur $A_s$ hinaus wird die Verformung nur zum Teil wieder aufgehoben und eine anschließende Abkühlung führt dann zu einer Rückverformung, die jedoch nicht vollständig an die ursprüngliche Verformung heranreicht. Durch Erwärmen und Abkühlen kann nun reversibel zwischen den beiden Verformungszuständen gewechselt werden (Zweiweg-Effekt). Nähere Einzelheiten hierzu sind beispielsweise aus dem Sonderdruck der "zeitschrift für wirtschaftliche Fertigung", 81. Jahrgang 1986, Heft 12, S. 203, von Dr. P. Tautzenberger und Prof. Dr. D. Stöckel: "Anwendung von Formgedächtnislegierungen in der Technik" bekannt.

Die maximale reversible Verformung die unter Außenlast erreichbar ist beträgt bei den bekannten Memory-Metallegierungen ca. 8% (Einweg-Effekt). Die mehrmalige Zyklierung der belasteten Drähte über den Umwandlungstemperaturbereich hinaus trainiert diese Drähte zum Zweiweg-Effekt, wobei die reversible Verformung bis zu 3 % während der Zyklierung über den Umwandlungstemperaturbereich spontan ohne Außenbelastung entsteht.

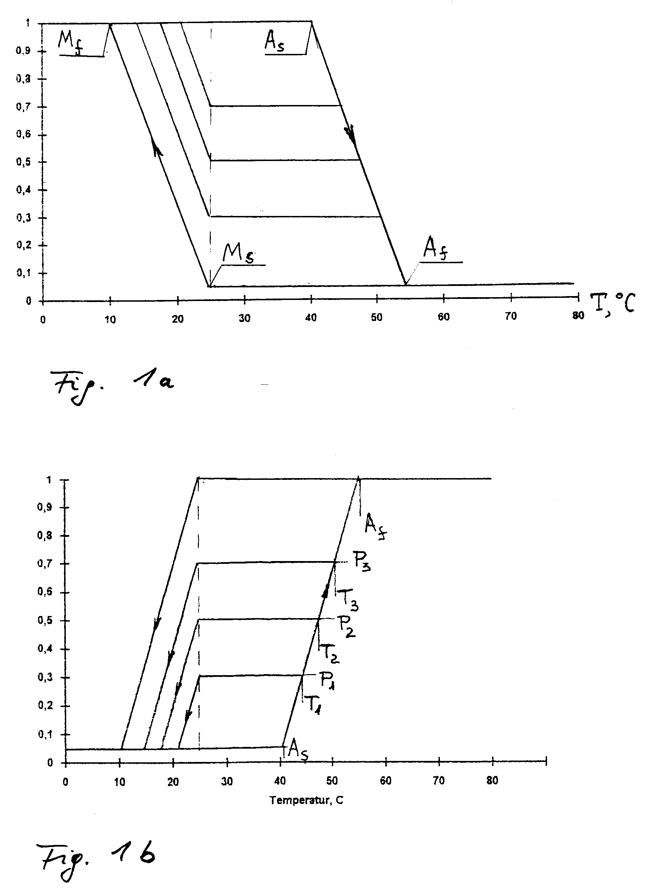

Figuren 1a und 1b zeigen schematisch eine idealisierte Hysteresekurve (Zweiweg-Effekt) von Memory-Metallegierungen, wobei in den Figuren 1a und 1b auf der x-Achse die Temperatur und auf der y-Achse in Fig. 1a die Länge bzw. Dehnung eines Drahtes aus Memory-Metall und in Fig. 1b der erreichte Druck mit einem Verbundgewebe gemäß der vorliegenden Erfindung in relativen Einheiten aufgetragen ist. Hierbei ist $M_s$ die Temperatur, bei der die martensitische Hinumwandlung bzw. Verformung bei Temperaturerhöhung beginnt, $M_f$ ist die Temperatur, bei der die martensitische Hinumwandlung endet bzw. die Verformung maximal wird, $A_s$ die Temperatur, bei der bei Temperaturerniedrigung die martensitische Rückumwandlung einsetzt und $A_f$ die Temperatur, bei der die Rückumwandlung endet.

In Fig. 1a wird ausgehend von einem Draht mit maximaler Länge (y=1) die Temperatur bis zu einer Temperatur $A_s$ erhöht, bei der die Kontraktion des Drahtes einsetzt. Die Kontraktion setzt sich fort (y wird kleiner), bis eine Temperatur $A_f$ erreicht ist. Bei weiterer Temperaturerhöhung tritt keine weitere Kontraktion auf. Wird nun der Memory-Draht abgekühlt, setzt bei einer Temperatur $M_s$, die kleiner als die Temperatur $A_s$ ist, die Rückumwandlung ein und der Draht beginnt sich wieder auszudehnen (y wird wieder größer). Diese Ausdehnung setzt sich fort bis idealerweise bei einer Temperatur $M_f$ der Ausgangszustand (y=1) wieder erreicht ist.

Fig. 1b zeigt eine analoge Darstellung, wobei lediglich auf der y-Achse der erzeugbare Druck in relativen Einheiten aufgetragen ist. Wie aus den Figuren 1a und 1b zu ersehen ist, kann durch Erhitzen auf eine bestimmte Temperatur, die unterhalb der jeweils maximal möglichen Temperatur liegt, das Ausmaß der Längenkontraktion und damit des applizierten Druckes eingestellt werden. D. h. es werden die inneren Hystereseschleifen durchfahren.

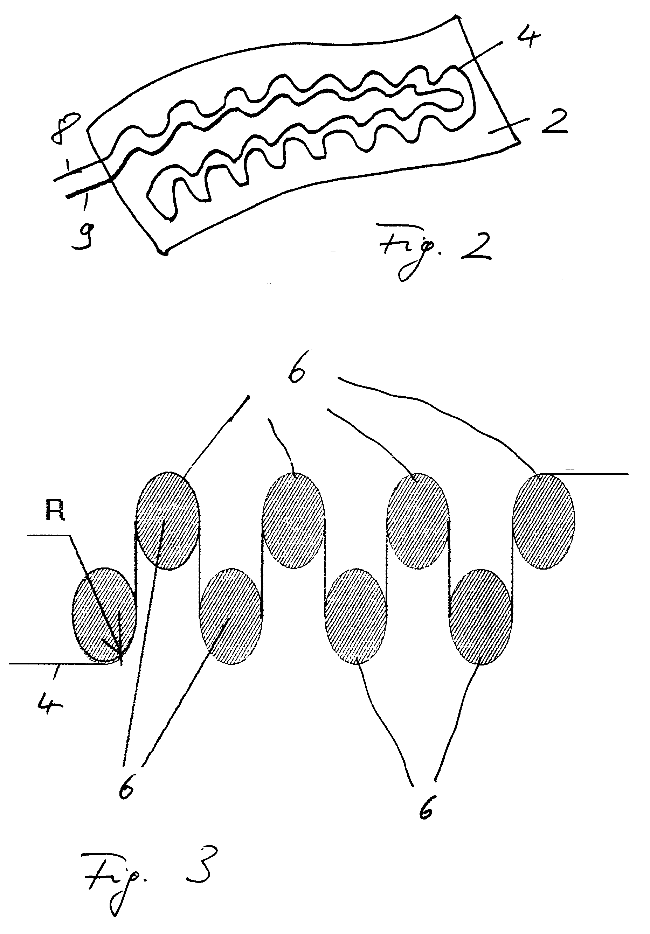

Fig. 2 zeigt eine erste Ausführungsform der Erfindung in Form eines flächigen Verbundgewebes, das ein flächiges, streifen- bzw. bandförmiges Stoffgewebe 2 und einen darin eingearbeiteten Memory-Draht 4 aufweist. Der Memory-Draht 4 ist mäanderförmig ausgebildet, wodurch die Längenänderung des Drahts bzw. des Stoffgewebes 2, in der der Memory-Draht 4 eingearbeitet ist, vervielfacht wird.

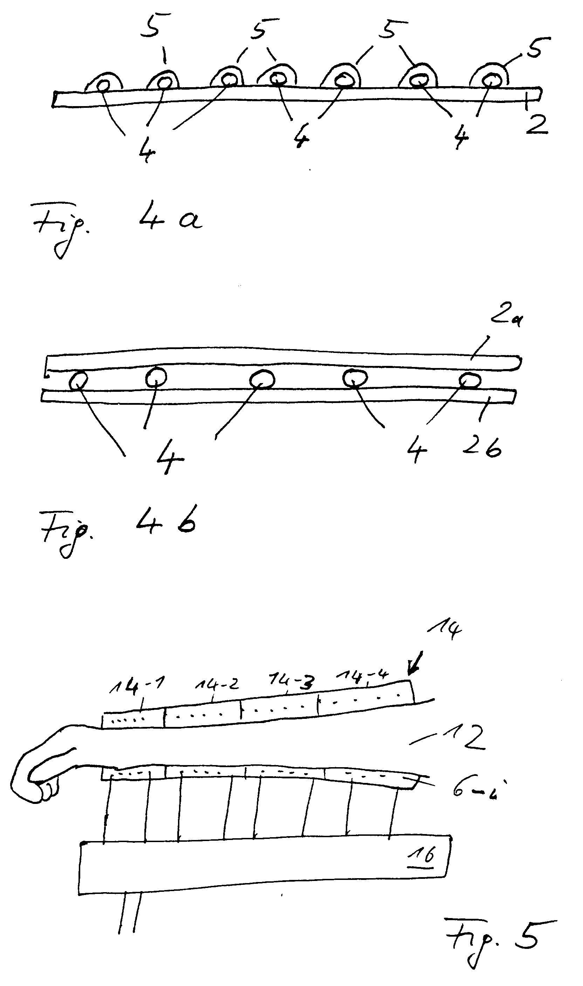

Die Verformung des Memory-Drahts 4 zu einer mäanderförmigen, flachen Feder erfolgt vorzugsweise mittels eines Biegewerkzeugs, wie es in Fig. 3 schematisch dargestellt ist. Wie aus Fig. 3 zu ersehen ist, wird der Memory-Draht 4 mäanderförmig um versetzt zueinander angeordnete abgerundete Biegestifte 6 gebogen. Hierbei stellt der Radius R den Krümmungsradius der Außenfläche der Biegestifte 6 dar und r stellt den Radius der Memory-Drähte 4 dar. Bei einer besonders vorteilhaften Ausgestaltung der Erfindung gilt hierbei das folgende Verhältnis:

$$r/R \times 100\% < 8\%$$

Der Memory-Draht 4 in der Ausführungsform gemäß Fig. 2 ist als Endlosdraht ausgeführt und endet in elektrischen Anschlüssen 8 und 9. An den elektrischen Anschlüssen 8 und 9 kann ein nicht näher dargestelltes elektrisches Stromversorgungsgerät angeschlossen werden, mittels dem der Memory-Draht 4 durch kurzzeitige Stromimpulse auf eine Temperatur T über der Temperatur $A_s$ erhitzt wird, was zu einer Kontraktion des Drahtes 4 und damit des Stoffgewebes 2 führt.

Wird beispielsweise das Verbundgewebe von Fig. 2 als Stützbandage um ein Knie verwendet, so wird zunächst das streifen- bzw. bandförmige Verbundgewebe nach Art eines herkömmlichen elastischen Verbands auf dem Knie appliziert und anschließend wird der in das Stoffgewebe 2 eingearbeitete Memory-Draht 4 mittels elektrischem Strom auf eine Temperatur T erhitzt, die über der Temperatur $A_s$ liegt.

Durch entsprechende Auswahl der Materialien bzw. der Zusammensetzung der Legierung für den Memory-Draht 4 kann die Temperatur $A_s$ so eingestellt werden, daß sie über der maximalen Körpertemperatur bzw. Umgebungstemperatur liegt. Damit wird beispielsweise erreicht, daß die elektrische Stromquelle nach dem Erhitzen des Memory-Drahtes 4 und damit dem Kontrahieren des Stoffgewebes 2 entfernt werden kann und dennoch die Stützfunktion bzw. die Kontraktion des Stoffgewebes 2 erhalten bleibt. Die Temperatur $M_f$, bei dem bei Abkühlung des Memory-Drahtes 4 die Längenausdehnung wieder einsetzt, ist hierbei so gewählt, daß diese kleiner als die Körpertemperatur bzw. die Umgebungstemperatur ist. Damit kann der Stütz- bzw. Pressverband auf einfache Weise wieder dadurch entfernt werden, daß der am Körper befindliche Verband beispielsweise mittels Eisbeutel abgekühlt wird.

Anstelle von einem einzigen Memory-Draht 4, wie dies beispielhaft in Fig. 2 dargestellt ist, lassen sich auch mehrere Memory-Drähte 4 in das Stoffgewebe 2 einarbeiten. Mehrere Memory-Drähte 4 lassen sich dann elektrisch parallel oder in Serie schalten. Auch ein getrennte elektrische Ansteuerung ist möglich.

Die charakteristischen Temperaturen $M_f$, $M_s$, $A_f$ und $A_s$, können durch geeignete Materialwahl und deren Zusammensetzung in gewissen Grenzen eingestellt werden. Legierungen mit Formgedächtniseigenschaften sind NiTi-, Cu-Zn-Al- und Cu-Al-Ni-Legierungen. Weitere Informationen zu geeigneten Materialien bzw. Legierungen sind beispielsweise aus der Zeitschrift METALL, 41. Jhargang, Heft 5, Mai 1987, Seiten 488 bis 493 bekannt.

Fig. 4a und 4b zeigen schematische Darstellungen, die bevorzugte Befestigungen des bzw. der Memory-Drähte 4 in bzw. an dem Stoffgewebe 2 zeigen. In der in Fig. 4a gezeigten Ausführungsform wird der bzw. die Memory-Drähte 4 auf das Stoffgewebe 2 mittels Steppfäden 5 aufgesteppt. Bei der in Fig. 4b gezeigten Ausführungsform umfaßt das Stoffgewebe 2 zwei Schichten 2a und 2b und der bzw. die Memory-Drähte 4 werden zwischen diesen beiden Schichten 2a und 2b eingeschweißt oder auch eingenäht. In beiden Fällen wird eine einfache Herstellung des erfindungsgemäßen Verbundgewebes dadurch erreicht, daß kein speziell hergestelltes Stoffgewebe notwendig ist, sondern die Memory-Drähte 4 werden auf oder zwischen herkömmliche im Handel erhältliche Stoffgewebe eingearbeitet.

Fig. 5 zeigt schematisch eine Massagevorrichtung bzw. eine Vorrichtung 10 zur intermittierenden Kompression bei der Behandlung von Ödemen, die beispielhaft an einem Arm 12 einer Person angeordnet ist. Die Massagevorrichtung 10 umfaßt eine Manschette 14 in die der Arm 12 eingeführt wird. In die Manschette 14 sind Memory-Drähte 4-1, 4-2, 4-3 und 4-4 eingearbeitet, die sich bei Temperaturerhöhung über die Temperatur $A_s$ zusammenziehen und somit eine Druckbeaufschlagung durch die Manschette 14 in etwa senkrecht zur Hautoberfläche des Arms 12 bewirken. Die Manschette 14 ist in vier aneinander angrenzende, schlauchförmige Bereiche 14-1, 14-2, 14-3 und 14-4 unterteilt. Die Bereiche 14-i werden jeweils von den Memory-Drähten 6-i durchsetzt. Die Memory-Drähte 6-i sind über elektrische Anschlüsse 8-i bzw. 9-i mit einer Drucksteuereinrichtung 16 verbunden.

Mittels der Drucksteuereinrichtung 16 lassen sich die einzelnen Memory-Drähte 6-i in den einzelnen Bereichen 14-i der Manschette 14 gezielt und unabhängig voneinander durch Stromimpulse erhitzen. Auf diese Weise ist es möglich eine Druckwelle aufzubauen, die beispielsweise ausgehend von dem Bereich 14-1 sich zu dem Bereich 14-4 fortpflanzt. Darüber hinaus ist es mit der erfindungsgemäßen Massageeinrichtung 10 möglich einen Druckgradienten, der beispielsweise dazu führt, daß der auf den Arm 12 ausgeübte Druck in dem Bereich 14-1 am höchsten ist und zum Bereich 14-4 hin abnimmt.

Arbeitet man die Memory-Drähte 6 in geeigneter Weise in einen Anzug ein, ergibt sich auf einfache Weise ein Druckanzug der sich elektrisch Ansteuern läßt und die komplizierte mit Druckluft arbeitende Druckkleidung von Piloten und Astronauten ersetzt.

**Patentansprüche**

1. Flächiges Verbundgewebe mit einem flächigen Stoffgewebe (2) und darin eingearbeiteten Fäden bzw. Drähten (4) aus einer Legierung mit Formgedächtniseigenschaften.

2. Verbundgewebe nach Anspruch 1, dadurch gekennzeichnet, daß die Drähte (4) aus einer Legierung mit Formgedächtniseigenschaften auf das flächige Stoffgewebe (2) aufgesteppt sind.

3. Verbundgewebe nach Anspruch 1, dadurch gekennzeichnet, daß das flächige Stoffgewebe (2) wenigstens zwei Schichten (2a, 2b) aufweist und daß die Drähte (4) aus einer Legierung mit Formgedächtniseigenschaften zwischen den wenigstens zwei Schichten des Stoffgewebes eingearbeitet sind.

4. Verbundgewebe nach wenigstens einem der vorhergehenden Ansprüche, dadurch gekennzeichnet, daß die Drähte (4) aus einer Legierung mit Formgedächtniseigenschaften sich bei Erwärmung über eine bestimmte Temperatur $A_s$ verkürzen und bei Abkühlung unter eine bestimmte Temperatur $M_s$ wieder ihre ursprüngliche Form annehmen (Zweiweg-Memory-Effekt).

5. Verbundgewebe nach wenigstens einem der vorhergehenden Ansprüche, dadurch gekennzeichnet, daß das Stoffgewebe (2) elastisch ist.

6. Verbundgewebe nach wenigstens einem der vorhergehenden Ansprüche, dadurch gekennzeichnet, daß die Drähte (4) aus einer Legierung mit Formgedächtniseigenschaften einen runden Querschnitt aufweisen.

7. Verbundgewebe nach wenigstens einem der vorhergehenden Ansprüche, dadurch gekennzeichnet, daß die Drähte (4) aus einer Legierung mit Formgedächtniseigenschaften in Form einer flachen Feder gebogen sind.

8. Verbundgewebe nach Anspruch 7, dadurch gekennzeichnet, daß die Drähte (4) aus einer Legierung mit Formgedächtniseigenschaften mäanderförmig sind, wobei für das Verhältnis des Radius R an den Biegestellen zu dem Radius des Drahtes gilt:

$$r/R \times 100\% < 8\%$$

9. Verbundgewebe nach wenigstens einem der vorhergehenden Ansprüche, dadurch gekennzeichnet, daß die Drähte (4) aus einer Legierung mit Formgedächtniseigenschaften unterschiedliche Querschnitte aufweisen.

10. Verbundgewebe nach wenigstens einem der vorhergehenden Ansprüche, dadurch gekennzeichnet, daß die Drähte (4) aus einer Legierung mit Formgedächtniseigenschaften Anschlüsse (8, 9) für eine Stromquelle aufweisen.

11. Verbundgewebe nach wenigstens einem der vorhergehenden Ansprüche, dadurch gekennzeichnet, daß die Drähte (4) aus einer Legierung mit Formgedächtniseigenschaften geschlossene Drahtschleifen umfassen.

12. Verbundgewebe nach wenigstens einem der vorhergehenden Ansprüche, dadurch gekennzeichnet, daß das Stoffgewebe (2) aus einem elektrisch isolierenden Material besteht.

13. Verbundgewebe nach wenigstens einem der vorhergehenden Ansprüche, dadurch gekennzeichnet, daß das Stoffgewebe (2) als Endlosverband bzw. Verbandsbinde ausgebildet ist.

14. Verbundgewebe nach wenigstens einem der vorhergehenden Ansprüche, dadurch gekennzeichnet, daß das Stoffgewebe (2) als Manschette (14) ausgebildet ist.

15. Verbundgewebe nach wenigstens einem der vorhergehenden Ansprüche, dadurch gekennzeichnet, daß die Drähte (4) aus einer Legierung mit Formgedächtniseigenschaften aus einer Cu-Al-X-Legierung bestehen, wobei X aus den Elementen Zn, Mn und Ni ausgewählt ist.

16. Preß- bzw. Stützverband mit einem Verbundgewebe nach wenigstens einem der vorhergehenden Ansprüche 3 bis 15, dadurch gekennzeichnet, daß die Temperatur $A_s$ größer als die durchschnittliche Temperatur des Körperteils ist, auf das der Preß- bzw. Stützverband aufgebracht wird.

17. Preß- bzw. Stützverband nach Anspruch 16, dadurch gekennzeichnet, daß die Temperatur $M_s$ kleiner als die durchschnittliche Temperatur des Körperteils ist, auf das der Preß- bzw. Stützverband aufgebracht wird.

18. Preß- bzw. Stützverband nach Anspruch 16 oder 17, dadurch gekennzeichnet, daß die Drähte (4) aus einer Legierung mit Formgedächtniseigenschaften mit einer elektrischen Stromquelle verbindbar sind.

19. Massagevorrichtung mit einer den zu massierenden Körperteil umschließenden Hülle bzw. Manschette (14) und einer Drucksteuereinrichtung (16) zum Erzeugen von unterschiedlichen Drücken in unterschiedlichen Bereichen (14-i) der Manschette (14), dadurch gekennzeichnet,

daß die Manschette (14) Verbundgewebe nach wenigstens einem der vorhergehenden Ansprüche 1 bis 15 umfaßt,
daß die Temperatur $A_s$ und die Temperatur $M_s$ der Legierung mit Formgedächtniseigenschaften größer als die durchschnittliche Temperatur des zu massierenden Körperteils ist, und
daß die Drucksteuereinrichtung (16) eine elektrische Steuereinrichtung zum Erzeugen von Stromimpulsen in den Drähten (4) aus einer Legierung mit Formgedächtniseigenschaften umfaßt.

20. Massagevorrichtung nach Anspruch 19, dadurch gekennzeichnet, daß unterschiedliche Bereiche (14-i) der Hülle bzw. Manschette (14) durch die Drucksteuereinrichtung (16) getrennt ansteuerbar sind.

21. Massagevorrichtung nach wenigstens einem der Ansprüche 19 bis 20, gekennzeichnet durch wenigstens einen Drucksensor in der Hülle bzw. Manschette (14) zum Erfassen des Massagedrucks.

22. Druckanzug für Piloten und Astronauten aus einem Stoffgewebe und einer Drucksteuereinrichtung zum Erzeugen von unterschiedlichen Drücken in unterschiedlichen Bereichen des Druckanzugs, dadurch gekennzeichnet, daß das Stoffgewebe eien Verbundgewebe nach wenigstens einem der Ansprüche 1 bis 14 umfaßt.

23. Druckanzug nach Anspruch 22, dadurch gekennzeichnet, daß die Drucksteuereinrichtung eine elektrische Steuereinrichtung zum Erzeugen von Stromimpulsen in den Drähten aus einer Legierung mit Formgedächtniseigenschaften umfaßt.

Fig. 1a

Temperatur, C

Fig. 1b

7

Fig. 2

Fig. 3

Fig. 4 a

Fig. 4 b

Fig. 5